# EUROPEAN PATENT SPECIFICATION

(11) **EP 4 358 865 B1**
(45) Date of publication and mention of the grant of the patent: **14.10.2026**
(21) Application number: 22740962.0
(22) Date of filing: 16.06.2022
(51) Int. Cl.: A61B 17/072

(54) **SURGICAL STAPLING DEVICE INCLUDING A BUTTRESS RETENTION ASSEMBLY**
CHIRURGISCHE KLAMMERVORRICHTUNG MIT VERSTEIFUNGSHALTEANORDNUNG
DISPOSITIF D'AGRAFAGE CHIRURGICAL COMPRENANT UN ENSEMBLE DE RETENUE DE RENFORT

(30) Priority: 24.06.2021 US 202117357363
(43) Date of publication of application: 01.05.2024
(73) Proprietor: Covidien LP, Mansfield, MA 02048 (US)
(72) Inventor: BARIL, Jacob C., Norwalk, Connecticut 06851 (US); THOMAS, Justin J., New Haven, Connecticut 06512 (US); BANERJEE, Saumya, North Haven, Connecticut 06473 (US)
(74) Representative: Zimmermann & Partner Patentanwälte mbB
(86) International application number: PCT/IB2022/055592
(87) International publication number: WO 2022/269419

(56) References cited:
- EP-A2- 2 462 880
- EP-A2- 3 150 142
- US-A1- 2021 177 415

## Description

### TECHNICAL FIELD

The disclosure relates to surgical stapling devices, and more particularly, to assemblies and methods for detachably securing or retaining a staple line buttress assembly to a surgical stapling device.

### BACKGROUND

Surgical stapling devices are employed by surgeons to sequentially or simultaneously apply one or more rows of fasteners, e.g., staples or two-part fasteners, to body tissue for the purpose of joining segments of body tissue together. When stapling relatively thin or fragile tissues, it is important to effectively seal the staple line against air or fluid leakage. Additionally, it is often necessary to reinforce the staple line against the tissue to inhibit tears in the tissue or pulling of the staples through the tissue. One method of inhibiting tears or pull through involves the placement of a biocompatible reinforcing material or "buttress" material, between the staples and the underlying tissue. In this method, a layer of buttress assembly is placed against the tissue and the tissue is stapled in the conventional manner.

Accordingly, new systems and methods that enable easy and efficient attachment and removal of a buttress assembly to the surgical stapling device would be desirable.

Documents EP 3 150 142 A2, US 2021/177415 A1, EP 2 462 880 A2 disclose surgical staplers with buttress assemblies.

### SUMMARY

The disclosure describes a surgical stapling device including a buttress retention assembly that demonstrates a practical approach to meeting the performance requirements and overcoming usability challenges associated with detachably securing a buttress assembly to a surgical stapling device.

In accordance with the invention and as defined in claim 1, a surgical stapling device includes a tool assembly and a buttress assembly. The tool assembly includes first and second jaw members that are transitionable between closed and open configurations. At least one of the first or second jaw members includes a retention assembly including a support defining a recess. The buttress assembly includes a buttress material and a spine. The buttress material includes first and second portions. The spine includes an elongate portion extending along a length of the first and second portions of the buttress material and an engaging portion attached to the elongate portion in an orthogonal relation. The engaging portion is detachably received in the recess of the support of the retention assembly of the at least one of the first or second jaw members.

According to the invention and as defined in claim 1, the buttress material includes a living hinge that connects the first and second portions of the buttress material to each other. The living hinge biases the first and second portions away from each other.

Specific embodiments are set forth in the dependent claims.

In another aspect, the first and second portions of the buttress material may define a V-shape profile.

In yet another aspect, the buttress material may be monolithically formed.

In still yet another aspect, the engaging portion of the spine may extend radially outwards from an end portion of the elongate portion of the spine.

In an aspect, the at least one of the first or second jaw members may have a tapered end, and the support of the retention assembly may be disposed on the tapered end.

In another aspect, the support of the retention assembly may have a triangular shape to reduce bending of the buttress material and the spine.

In yet another aspect, the elongate portion of the spine may extend along a peripheral portion of the buttress material.

In still yet another aspect, the engaging portion of the buttress assembly may be releasably secured to the support of the retention assembly by snap fit or friction fit.

In still yet another aspect, the spine of the buttress material may be bioabsorbable.

In still yet another aspect, at least a portion of the elongate portion of the spine may be interposed between the buttress material and the first or second jaw members.

In accordance with another aspect of the disclosure, a surgical kit includes a buttress assembly, a loading assembly, and a surgical stapling device. The buttress assembly includes a buttress material and a first spine. The buttress material includes first and second portions. The first spine includes an elongate portion extending along a length of the first and second portions of the buttress material and engaging portions extending laterally outwards from respective distal end portions of the elongate portion. The loading assembly includes a base portion and an extension extending from the base portion. The base portion includes first and second anchoring assemblies on opposite ends of the base portion. The buttress assembly includes a portion that is wrapped around the extension of the loading assembly. The engaging portions of the first spine of the buttress assembly is detachably secured to the respective first and second anchoring assemblies of the base portion of the loading assembly. The surgical stapling device includes a tool assembly having first and second jaw members that are transitionable between closed and open configurations. The first or second jaw members includes respective retention assemblies. Each retention assembly includes a support defining a recess configured to releasably receive a corresponding engaging portion of the first spine.

In an aspect, the buttress material may further include a living hinge interconnecting the first and second portions of the buttress material. The living hinge may bias the first and second portions away from each other.

In another aspect, the buttress material may be monolithically formed as a single construct.

In yet another aspect, the engaging portions of the first spine may extend from the respective distal end portions of the elongate portion in an orthogonal relation.

In still yet another aspect, the buttress assembly supported on the loading assembly may define a first angle. The first and jaw members may define a second angle in the spaced apart configuration. The first and second angles may be equal.

In still yet another aspect, the buttress assembly may further include a second spine. The first and second spine may be laterally spaced apart and disposed on opposite lateral sides of the buttress material.

In an aspect, the retention assembly of the first or second jaw members may include a pair of supports defining recesses.

In another aspect, the first or second spines may be formed of a bioabsorbable material.

In yet another aspect, the support of the retention assembly of the surgical stapling device may secure the spine thereto by snap fit or interference fit.

### BRIEF DESCRIPTION OF DRAWINGS

The above and other aspects and features of this disclosure will become more apparent in view of the following detailed description when taken in conjunction with the accompanying drawings wherein like reference numerals identify similar or identical elements.
FIG. 1 is a perspective view of a surgical stapling device in accordance with the disclosure;
FIG. 2 is a perspective view of a buttress assembly of FIG. 1 for use with the surgical stapling device;
FIG. 3 is an enlarged view of the indicated area of detail of FIG. 1;
FIG. 4 is a partial side view of the surgical stapling device of FIG. 1, illustrating the buttress assembly secured to jaws of a tool assembly of the surgical stapling device;
FIG. 5 is an enlarged view of the indicated area of detail of FIG. 4;
FIG. 6 is a perspective view of a loading assembly for supporting the buttress assembly of FIG. 2 prior to use with the surgical stapling device of FIG. 1;
FIG. 7 is an enlarged view of the indicated area of detail of FIG. 6;

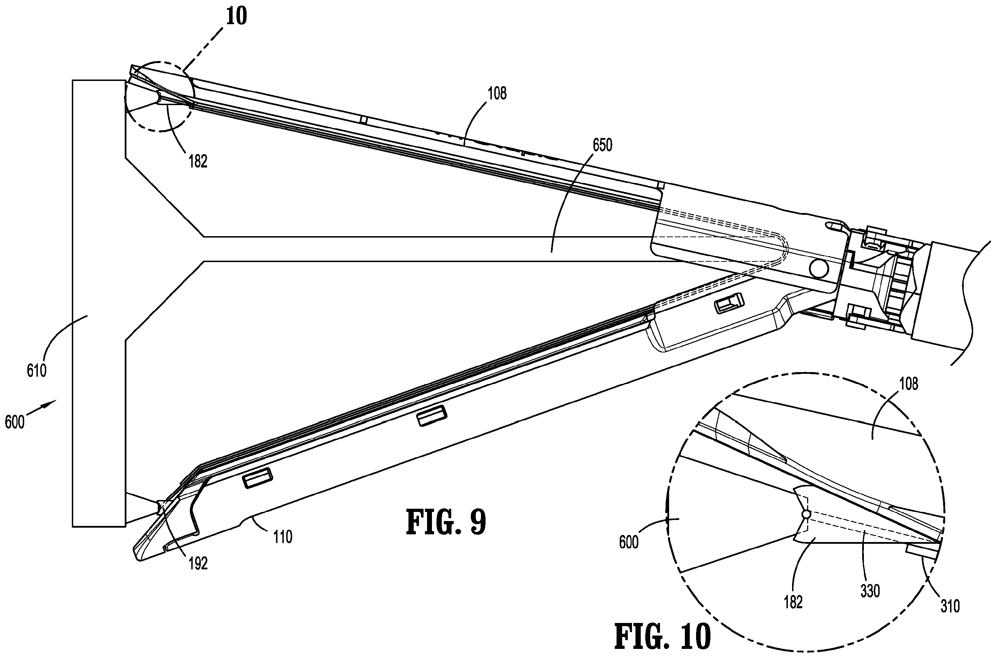

FIGS. 8 and 9 are partial side views of the surgical stapling device of FIG. 1 and the loading assembly of FIG. 6, illustrating transfer of the buttress assembly from the loading assembly to the surgical stapling device; and
FIG. 10 is an enlarged view of the indicated area of detail of FIG. 9.

### DETAILED DESCRIPTION

The surgical stapling device including a buttress retention assembly disclosed herein is described in detail with reference to the drawings, in which like reference numerals designate identical or corresponding elements in each of the several views. As used herein, the term "distal" refers to the portion that is being described which is farther from a user in a conventional use of the surgical stapling device, while the term "proximal" refers to the portion that is being described which is closer to a user in a conventional use of the surgical stapling device. In addition, the terms parallel and perpendicular are understood to include relative configurations that are substantially parallel and substantially perpendicular up to about + or - 10 degrees from true parallel and true perpendicular. Further, to the extent consistent, any or all of the aspects detailed herein may be used in conjunction with any or all of the other aspects detailed herein.

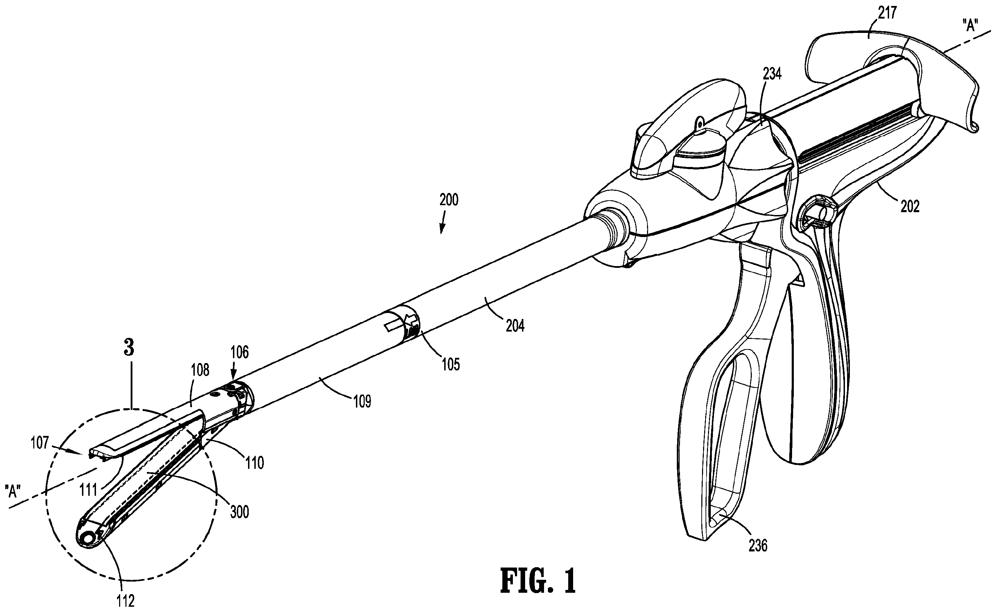

With reference to FIG. 1, there is provided a surgical stapling device 200 for use in stapling tissue and applying a layer of buttress material between staples and underlying tissue. The layer of buttress material is configured to reinforce and seal staple lines applied to tissue by the surgical stapling device 200. The surgical stapling device 200 generally includes a handle 202 and an elongate tubular member 204 extending distally from the handle 202. A reload 106 is removably coupled to a distal end 105 of the elongate tubular member 204. The reload 106 includes a shaft portion 109 and a tool assembly 107 supported on the shaft portion 109. The tool assembly 107 includes first jaw member 108 and a second jaw member 110 that is movable in relation to the first jaw member 108 between an open configuration for positioning tissue between the first and second jaw members 108, 110 and a closed configuration for clamping tissue between the first and second jaw members 108, 110 and subsequently stapling tissue. The first jaw member 108 supports an anvil 111 and the second jaw member 110 releasably supports a staple cartridge 112. In order to secure the staples provided by the staple cartridge 112 to tissue and a buttress assembly 300, the anvil 111 is provided with longitudinally arranged rows of staple clinching or forming pockets (not shown). It is envisioned that the tool assembly 107 may be coupled to a mechanical or motorized handle, and the staple cartridge 112 may be removable and replaceable. It is also envisioned that the reload 106 may be part of a robotic surgical system.

With continued reference to FIG. 1, the surgical stapling device 200 includes a trigger 236 movably mounted on the handle 202. Actuation of the trigger 236 transitions the tool assembly 107 from the open configuration to the closed configuration and subsequently actuates the surgical stapling device 200 to apply lines of staples to tissue. The surgical stapling device 200 further includes a retraction mechanism 217 that can be manually grasped and pulled proximally to retract a firing mechanism of the surgical stapling device 200. In order to provide proper orientation of the tool assembly 107 relative to tissue to be stapled, the surgical stapling device 200 is additionally provided with a rotation knob 234 mounted on the handle 202. Rotation of the rotation knob 234 about a longitudinal axis "A-A" of the surgical stapling device 200 rotates the tool assembly 107 about the longitudinal axis "A-A." Reference may be made to U.S. Patent Application Publication No. 2014/0263550, for a detailed discussion of the construction and operation of the surgical stapling device 200.

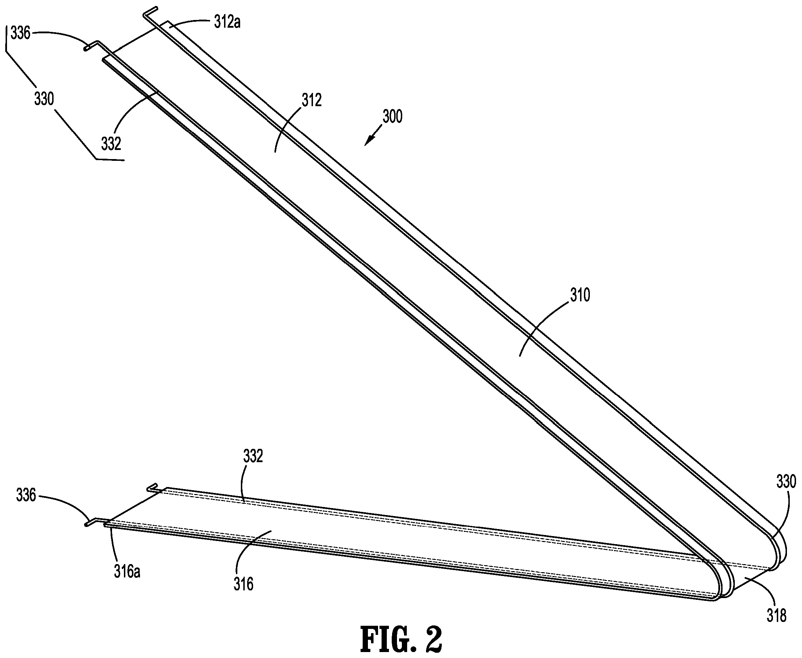

FIGS.1 and 2 illustrate a buttress assembly 300 for use with the surgical stapling device 200. The buttress assembly 300 is detachably secured to the tool assembly 107 of the surgical stapling device 200 to be in registration with the anvil 111 of the first jaw member 108 and the staple cartridge 112 of the second jaw member 110. The buttress assembly 300 includes a buttress material 310 and spines 330. The buttress material 310 is configured to reinforce and seal staple lines applied to tissue by the surgical stapling device 200. The buttress material 310 includes first and second portions 312, 316 that define a living hinge 318 therebetween. The living hinge 318 biases the first and second portions 312, 316 away from each other. In an aspect, the buttress material 310 has a V-shape. In an aspect, the buttress material 310 is monolithically formed as a single construct. In another aspect, the buttress material 310 may be formed of an elastic material. The spines 330 are laterally spaced apart and extend along opposing peripheral portions of the buttress material 310. In aspects, the spines 330 are provided to detachably secure the buttress assembly 300 to the first and second jaw members 108, 110 and maintain substantially planar surfaces of the first and second portions 312, 316 of the buttress material 310. Each spine 330 includes an elongate portion 332 that extends along the length of the buttress material 310 and engaging portions 336 that are disposed at opposite ends of the elongate portion 332. Each engaging portion 336 extends laterally outwards from the elongate portion 332 such that the engaging portion 336 is, e.g., orthogonal, to the elongate portion 332. The engaging portion 336 is detachably engageable with the tool assembly 107 (FIG. 1) of the surgical stapling device 200, as will be described. In particular, the elongate portion 332 extends distally from the distal ends 312a, 316a of the first and second portions 312, 316 of the buttress material 310 such that the engaging portions 336 are spaced apart from the distal ends 312a, 316a of the first and second portions 312, 316 of the buttress material 310. In an aspect, each spine 330 is monolithically formed as a single construct. In another aspect, the spines 330 may be formed of a polymer. In another aspect, the spines 330 may be bioabsorbable.

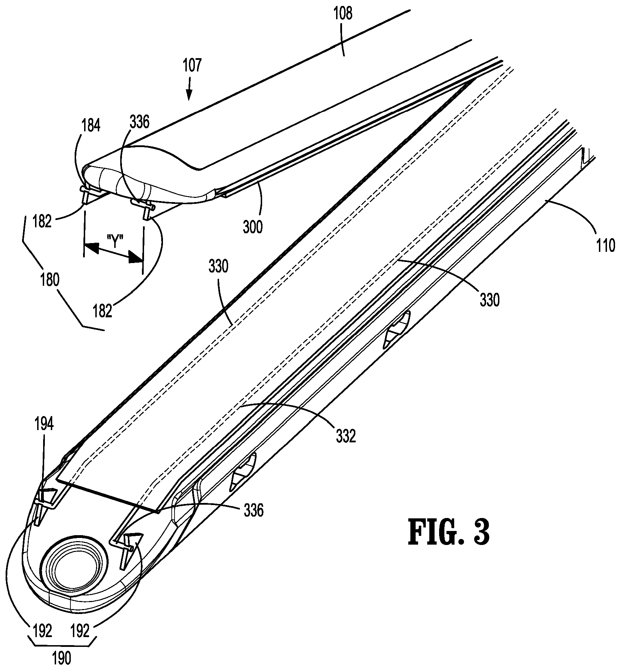

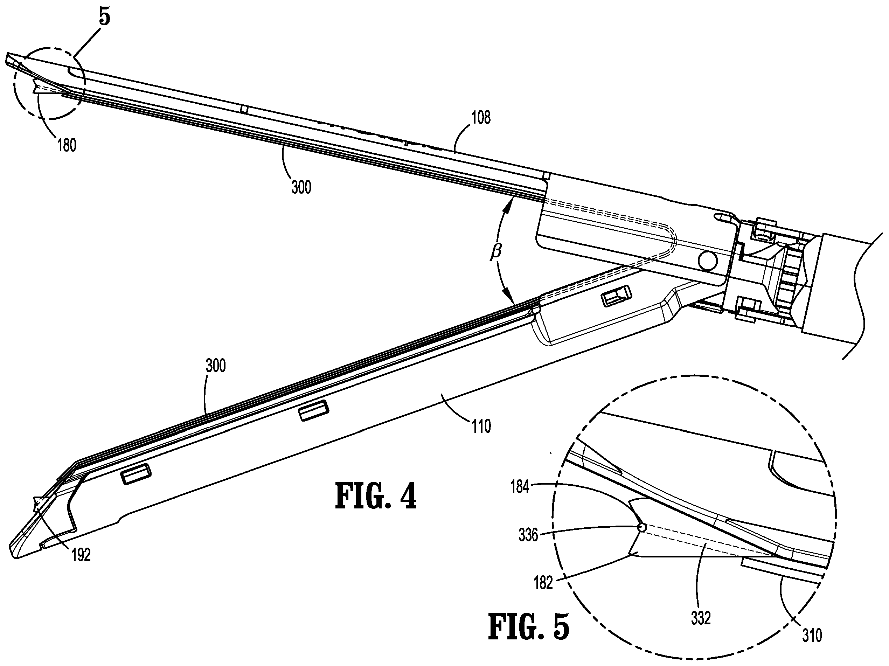

The buttress assembly 300 is detachably securable to the tool assembly 107. To this end, the first and second jaws members 108, 110 include respective first and second retention assemblies 180, 190, as shown in FIGS. 3 and 4. The first retention assembly 180 includes a pair of supports 182. Each support 182 defines a recess 184 to receive a corresponding engaging portion 336 of the spine 330. In an aspect, the engaging portion 336 may be detachably received in the recess 184 via, e.g., snap fit or interference fit. In an aspect, each support 182 of the first retention assembly 180 may include, e.g., a triangular, shape to compensate for the tapering of a distal end portion of the first jaw 108 in order to reduce bending of the spines 330 and/or the buttress material 310, as best shown in FIGS. 4 and 5. FIGS. 3 and 4 further illustrate the second jaw member 110 including the second retention assembly 190. Similar to the first retention assembly 180, the second retention assembly 190 includes a pair of supports 192. Each support 192 defines a recess 194 to receive a corresponding engaging portion 336 of the spine 330. In an aspect, the engaging portion 336 may be detachably received in the recess 194 via, e.g., snap fit or interference fit. In an aspect, each support 192 may include, e.g., a triangular, shape to compensate for the tapering of a distal end portion of the second jaw 110 in order to reduce bending of the spines 330 and/or the buttress material 310, as best shown in FIG. 4.

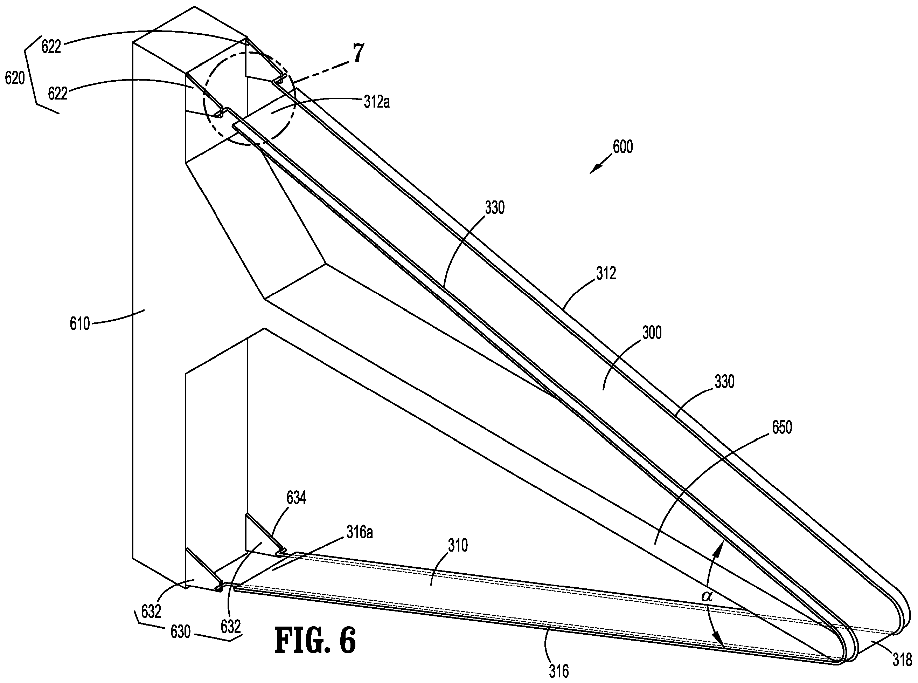

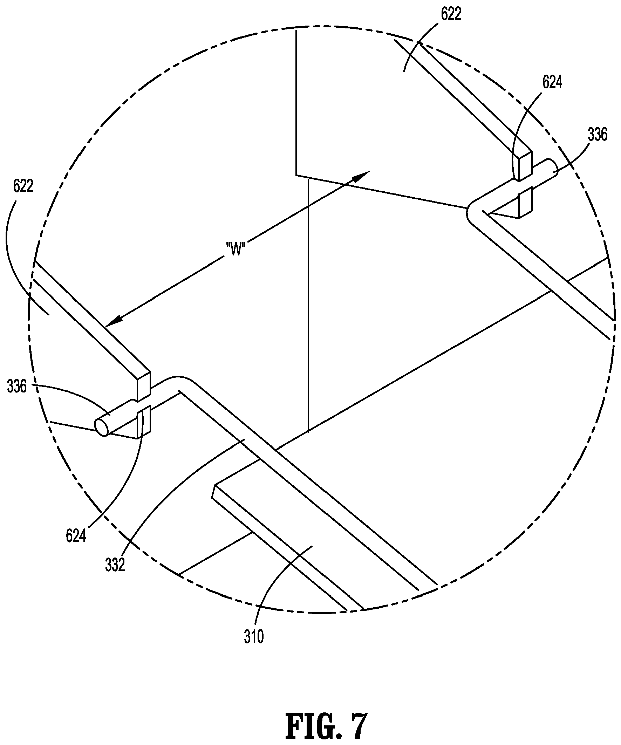

FIGS. 6 and 7 illustrate a loading assembly 600 releasably supporting the buttress assembly 300 prior to mounting the buttress assembly 300 onto the surgical stapling device 200. The loading assembly 600 facilitates mounting of the buttress assembly 300 on the tool assembly 107 of the surgical stapling device 200. The loading assembly 600 includes a base portion 610 and an extension 650 extending from the base portion 610. In particular, the base portion 610 includes anchoring portions 620, 630 on respective end portions of the base portion 610. The anchoring portions 620, 630 include a respective pair of supports 622, 632 that defines recesses 624 to detachably receive the engaging portions 336 of the spines 330 associated with the first portion 312 of the buttress assembly 300. In an aspect, the engaging portions 336 may be supported in the recesses 624 via, e.g., friction fit. Similarly, the pair of supports 632 of the anchoring portion 630 defines recesses 634 to detachably receive the engaging portions 336 of the spines 330 associated with the second portion 316 the buttress assembly 300. In an aspect, the supports 622 or supports 632 may be spaced apart by a distance "W" that is smaller or greater than a distance "Y" (FIG. 3) defined by the supports 182 or supports 192 of the first or second retention assemblies 180, 190 of the tool assembly 107 such that the first or second retention assemblies 180, 190 may be received within or laterally outwardly of the supports 622 or 632.

Under such a configuration, the buttress assembly 300 is wrapped around the extension 650, and the distal ends 312a, 316a of the buttress material 310 are detachably secured to the respective anchoring portions 620, 630. When the buttress assembly 300 is supported on the loading assembly 600, the first and second portions 312, 316 of the buttress assembly 300 define an angle α (FIG. 6) that is substantially identical to an angle β (FIG. 4) defined by the first and second jaw members 108, 110 in the open configuration.

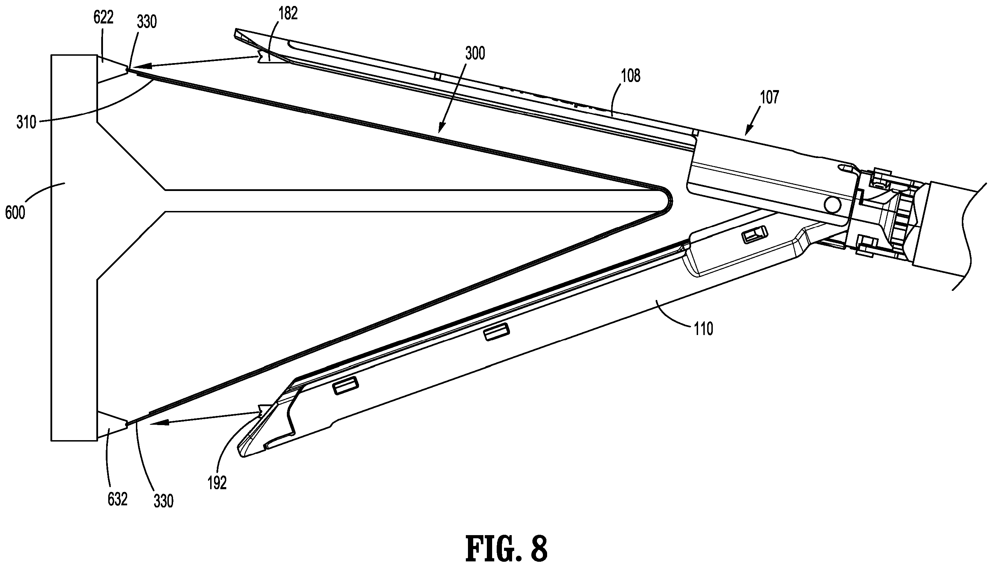

As shown in FIG. 8, prior to use, the buttress assembly 300 is detachably supported on the loading assembly 600. In use, the loading assembly 600 is positioned between the first and second jaw members 108, 110 with the jaw members 108, 110 in the open configuration. Thereafter, the supports 182, 192 of the first and second retention assemblies 180, 190 of the first and second jaw members 108, 110 are aligned with the supports 622, 632 of the loading assembly 600. The tool assembly 107 and the loading assembly 600 are further moved towards each other until the engaging portions 336 (FIG. 2) of the buttress assembly 300 are received in the recesses 184, 194 (FIGS. 3 and 5) of the first and second retention assemblies 180, 190. In this manner, the buttress assembly 300 is releasably mounted on the tool assembly 107 of the surgical stapling device 200. After the staples have been applied to the tissue, the buttress assembly 300 is attached to tissue and the buttress assembly 300 may be detached from the first and second retention assemblies 180, 190 by pulling the tool assembly 107 away from tissue.

It is further contemplated that the buttress assembly 300 may be made from any biocompatible natural or synthetic material. The material from which the buttress assembly 300 is formed may be bioabsorbable or non-bioabsorbable. It should be understood that any combination of natural, synthetic, bioabsorbable and non-bioabsorbable materials may be used to form the buttress assembly 300.

Some non-limiting examples of materials from which the buttress assembly 300 may be made include but are not limited to poly(lactic acid), poly (glycolic acid), poly (hydroxybutyrate), poly (phosphazine), polyesters, polyethylene glycols, polyethylene oxides, polyacrylamides, polyhydroxyethylmethylacrylate, polyvinylpyrrolidone, polyvinyl alcohols, polyacrylic acid, polyacetate, polycaprolactone, polypropylene, aliphatic polyesters, glycerols, poly(amino acids), copoly (ether-esters), polyalkylene oxalates, polyamides, poly (iminocarbonates), polyalkylene oxalates, polyoxaesters, polyorthoesters, polyphosphazenes and copolymers, block copolymers, homopolymers, blends and combinations thereof.

In aspects, natural biological polymers are used in forming the buttress assembly 300. Suitable natural biological polymers include, but are not limited to, collagen, gelatin, fibrin, fibrinogen, elastin, keratin, albumin, hydroxyethyl cellulose, cellulose, hydroxypropyl cellulose, carboxyethyl cellulose, chitan, chitosan, and combinations thereof. In addition, the natural biological polymers may be combined with any of the other polymeric materials described herein to produce the buttress assembly 300.

The buttress assembly 300 may be porous or non-porous, or combinations of porous and non-porous layers. Where the buttress assembly 300 is non-porous, the buttress assembly 300 may retard or inhibit tissue ingrowth from surrounding tissues thereby acting as an adhesion barrier and inhibiting the formation of unwanted scar tissue. Thus, in aspects, the buttress assembly 300 possesses anti-adhesion properties. Techniques for forming non-porous layers from such materials are within the purview of those skilled in the art and include, for example, casting, molding, and the like.

In aspects, the buttress assembly 300 is porous and possesses hemostatic properties. Where the buttress assembly 300 is porous, it has openings or pores over at least a portion of a surface thereof. Suitable materials for forming the porous layer include, but are not limited to foams (e.g., open or closed cell foams). In aspects, the pores may be in sufficient number and size so as to interconnect across the entire thickness of the porous layer. In other aspects, the pores do not interconnect across the entire thickness of the porous layer. In yet other aspects, the pores do not extend across the entire thickness of the porous layer, but rather are present at a portion of the surface thereof. In aspects, the openings or pores are located on a portion of the surface of the porous layer, with other portions of the porous layer having a non-porous texture. Those skilled in the art reading the disclosure will envision other pore distribution patterns and configurations for the porous layer.

Where the buttress assembly 300 is porous, the pores may be formed using any method suitable to forming a foam or sponge including, but not limited to the lyophilization or freeze-drying of a composition. Suitable techniques for making foams are within the purview of those skilled in the art. Porous buttress assembly 300 can be at least 0.2 cm thick, in aspects from about 0.3 to about 1.5 cm thick. Porous buttress assembly 300 can have a density of not more than about 75 mg/cm² and, in aspects below about 20 mg/cm². The size of the pores in the porous buttress assembly 300 can be from about 20 µm to about 300 µm, and in certain aspects from about 100 µm to about 200 µm.

The buttress assembly 300 may also include a reinforcement member. The reinforcement member may be associated with a porous or non-porous layer or may be positioned between a non-porous layer and a porous layer of the buttress assembly 300. Alternatively, the reinforcement member may be positioned entirely within one or more of the individual layers (e.g., embedded within the porous layer, the non-porous layer, or both) of the buttress assembly 300. It is also envisioned that the reinforcement member may be positioned at the surface of one of the layers making up the buttress assembly 300 and, in aspects, may be positioned at an exterior surface of the buttress assembly 300.

Some suitable non-limiting examples of reinforcement members include fabrics, meshes, monofilaments, multifilament braids, chopped fibers (sometimes referred to in the art as staple fibers) and combinations thereof. Where the reinforcement member is a mesh, it may be prepared using any technique known to those skilled in the art, such as knitting, weaving, tatting, knipling, or the like. Where monofilaments or multifilament braids are used as the reinforcement member, the monofilaments or multifilament braids may be oriented in any desired manner. For example, the monofilaments or multifilament braids may be randomly positioned with respect to each other within the buttress assembly 300. As another example, the monofilaments or multifilament braids may be oriented in a common direction within the buttress assembly 300. Where chopped fibers are used as the reinforcement member, the chopped fibers may be oriented in any desired manner. For example, the chopped fibers may be randomly oriented or may be oriented in a common direction. The chopped fibers can thus form a non-woven material, such as a mat or a felt. The chopped fibers may be joined together (e.g., by heat fusing) or they may be unattached to each other. The chopped fibers may be of any suitable length. For example, the chopped fibers may be from 0.1 mm to 100 mm in length, and in some aspects, 0.4 mm to 50 mm in length. In an aspect, the buttress assembly 300 has randomly oriented chopped fibers that have not been previously fused together and are embedded within in the buttress assembly 300.

It is envisioned that the reinforcement member may be formed from any bioabsorbable, non-bioabsorbable, natural, or synthetic material previously described herein and combinations thereof. Where monofilaments or multifilament braids are used as the reinforcement member, any commercially available suture material may advantageously be employed as the reinforcement member.

In aspects, at least one bioactive agent may be combined with the buttress assembly 300 and/or any of the individual components (the porous layer, the non-porous layer and/or the reinforcement member) used to construct the buttress assembly 300. In aspects, the buttress assembly 300 can also serve as a vehicle for delivery of the bioactive agent. The term "bioactive agent", as used herein, is used in its broadest sense and includes any substance or mixture of substances that have clinical use. Consequently, bioactive agents may or may not have pharmacological activity per se, e.g., a dye, or fragrance. Alternatively, a bioactive agent could be any agent which provides a therapeutic or prophylactic effect such as a compound that affects or participates in tissue growth, cell growth, or cell differentiation.

Examples of classes of bioactive agents which may be utilized in accordance with the disclosure include anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Anti-adhesive or anti-adhesion agents can be used to inhibit adhesions from forming between the buttress assembly 300 and the surrounding tissues opposite the target tissue. Some examples of these agents include, but are not limited to poly (vinyl pyrrolidone), carboxymethyl cellulose, hyaluronic acid, polyethylene oxide, poly vinyl alcohols and combinations thereof.

Suitable antimicrobial agents which may be included as a bioactive agent in the buttress assembly 300 of the disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent in the bioactive coating of the disclosure.

Other bioactive agents which may be included as a bioactive agent in the buttress assembly 300 in accordance with the disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; antispasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included in the coating composition include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; polynucleotides; and ribozymes.

While the disclosure has been shown in the drawings, it is not intended that the disclosure be limited thereto, as it is intended that the disclosure be as broad in scope as the art will allow and that the specification be read likewise. Therefore, the above description should not be construed as limiting, but merely as exemplifications.

## Claims

1. A surgical stapling device (200) comprising:
a tool assembly (107) including first and second jaw members (108, 110) that are transitionable between closed and open configurations, at least one of the first or second jaw members including a retention assembly (180, 190) including a support (182, 192) defining a recess (184, 194); and
a buttress assembly (300) including:
a buttress material (310) including first and second portions (312, 316),
wherein
the buttress assembly (300) further comprises a spine (330) including an elongate portion (332) extending along a length of the first and second portions (312, 316) of the buttress material (310) and an engaging portion (336) attached to the elongate portion (332) in an orthogonal relation, the engaging portion (336) detachably received in the recess (184, 194) of the support (182, 192) of the retention assembly (180, 190) of the at least one of the first or second jaw members (108, 110) and
**characterised in that** the buttress material (310) includes a living hinge (318) that connects the first and second portions of the buttress material to each other, the living hinge (318) biasing the first and second portions (312, 316) away from each other.

2. The surgical stapling device according to claim 1, wherein the first and second portions (312, 316) of the buttress material (310) defines a V-shape profile.

3. The surgical stapling device according to claim 1 or 2, wherein the buttress material (310) is monolithically formed.

4. The surgical stapling device according to any preceding claim, wherein the engaging portion (336) of the spine (330) extends radially outwards from an end portion of the elongate portion (332) of the spine, preferably wherein the at least one of the first or second jaw members (108, 110) has a tapered end, and the support (182, 192) of the retention assembly (180, 190) is disposed on the tapered end, more preferably wherein the support (182, 192) of the retention assembly (180, 190) has a triangular shape to reduce bending of the buttress material (310) and the spine (330).

5. The surgical stapling device according to any preceding claim, wherein the elongate portion of the spine (332) extends along a peripheral portion of the buttress material (310).

6. The surgical stapling device according to any preceding claim, wherein the engaging portion (336) of the buttress assembly (300) is releasably secured to the support (182, 192) of the retention assembly (180, 190) by snap fit or friction fit.

7. The surgical stapling device according to any preceding claim, wherein the spine (330) of the buttress assembly (300) is bioabsorbable.

8. The surgical stapling device according to any preceding claim, wherein at least a portion of the elongate portion (332) of the spine (330) is interposed between the buttress material (310) and the first or second jaw members (108, 110).

## Patentansprüche

1. Chirurgische Klammervorrichtung (200) umfassend:
eine Werkzeuganordnung (107), die ein erstes und ein zweites Backenelement (108, 110) aufweist, die zwischen einer geschlossenen und einer offenen Konfiguration überführbar sind, wobei wenigstens eines von dem ersten und dem zweiten Backenelement eine Rückhalteanordnung (180, 190) aufweist, die eine Träger (182, 192), der eine Vertiefung (184, 194) definiert, aufweist; und
eine Widerlageranordnung (300) aufweisend:
ein Widerlagermaterial (310), das einen ersten und einen zweiten Abschnitt (312, 316) aufweist,
wobei die Widerlageranordnung (300) ferner ein Rückgrat (330) umfasst, das einen langgestreckten Abschnitt (332), der entlang einer Länge des ersten und zweiten Abschnitts (312, 316) des Widerlagermaterials (310) verläuft, und einen Eingriffsabschnitt (336), der in einer orthogonalen Beziehung an dem langgestreckten Abschnitt (332) angebracht ist, aufweist, wobei der Eingriffsabschnitt (336) lösbar in der Vertiefung (184, 194) des Trägers (182, 192) der Rückhalteanordnung (180, 190) des wenigstens einen von dem ersten und dem zweiten Backenelements (108, 110) aufgenommen wird, und **dadurch gekennzeichnet, dass** das Widerlagermaterial (310) ein Filmscharnier (318) aufweist, das den ersten und den zweiten Abschnitt des Widerlagermaterials miteinander verbindet, wobei das Filmscharnier (318) den ersten und den zweiten Abschnitt (312, 316) voneinander weg gerichtet vorspannt.

2. Chirurgische Klammervorrichtung nach Anspruch 1, wobei der erste und der zweite Abschnitt (312, 316) des Widerlagermaterials (310) ein V-förmiges Profil definieren.

3. Chirurgische Klammervorrichtung nach Anspruch 1 oder 2, wobei das Widerlagermaterial (310) monolithisch gebildet ist.

4. Chirurgische Klammervorrichtung nach einem der vorstehenden Ansprüche, wobei der Eingriffsabschnitt (336) des Rückgrats (330) von einem Endabschnitt des langgestreckten Abschnitts (332) des Rückgrats radial nach außen vorsteht, wobei vorzugsweise das wenigstens eine von dem ersten und dem zweiten Backenelement (108, 110) ein verjüngtes Ende aufweist, und der Träger (182, 192) der Rückhalteanordnung (180, 190) an dem verjüngten Ende angeordnet ist, wobei bevorzugter der Träger (182, 192) der Rückhalteanordnung (180, 190) eine dreieckige Form aufweist, um Biegen des Widerlagermaterials (310) und des Rückgrats (330) zu verringern.

5. Chirurgische Klammervorrichtung nach einem der vorstehenden Ansprüche, wobei der langgestreckte Abschnitt des Rückgrats (332) entlang eines Randabschnitts des Widerlagermaterials (310) verläuft.

6. Chirurgische Klammervorrichtung nach einem der vorstehenden Ansprüche, wobei der Eingriffsabschnitt (336) der Widerlageranordnung (300) durch Schnappsitz oder Reibsitz lösbar an dem Träger (182, 192) der Rückhalteanordnung (180, 190) befestigt ist.

7. Chirurgische Klammervorrichtung nach einem der vorstehenden Ansprüche, wobei das Rückgrat (330) der Widerlageranordnung (300) bioabsorbierbar ist.

8. Chirurgische Klammervorrichtung nach einem der vorstehenden Ansprüche, wobei wenigstens ein Abschnitt des langgestreckten Abschnitts (332) des Rückgrats (330) zwischen dem Widerlagermaterial (310) und dem ersten oder zweiten Backenelement (108, 110) angeordnet ist.

## Revendications

1. Dispositif d'agrafage chirurgical (200) comprenant :
un ensemble outil (107) comprenant des premier et second éléments de mâchoire (108, 110) pouvant passer d'une configuration fermée à une configuration ouverte, au moins un des premier ou second éléments de mâchoire comprenant un ensemble de retenue (180, 190) comprenant un support (182, 192) définissant un évidement (184, 194) ; et
un ensemble de renfort (300) comprenant :
un matériau de renfort (310) comprenant des première et seconde parties (312, 316),
l'ensemble de renfort (300) comprenant en outre une structure (330) comprenant une partie allongée (332) s'étendant le long des première et seconde parties (312, 316) du matériau de renfort (310) et une partie de mise en prise (336) fixée à la partie allongée (332) dans une relation orthogonale, la partie de mise en prise (336) étant reçue de manière amovible dans l'évidement (184, 194) du support (182, 192) de l'ensemble de retenue (180, 190) de l'au moins un des premier ou second éléments de mâchoire (108, 110) et **caractérisé en ce que** le matériau de renfort (310) comprend une charnière mobile (318) qui relie les première et seconde parties du matériau de renfort l'une à l'autre, la charnière mobile (318) écartant les première et seconde parties (312, 316) l'une de l'autre.

2. Dispositif d'agrafage chirurgical selon la revendication 1, les première et seconde parties (312, 316) du matériau de renfort (310) définissant un profil en forme de V.

3. Dispositif d'agrafage chirurgical selon la revendication 1 ou 2, le matériau de renfort (310) étant formé de manière monolithique.

4. Dispositif d'agrafage chirurgical selon l'une quelconque des revendications précédentes, la partie de mise en prise (336) de la structure (330) s'étendant radialement vers l'extérieur à partir d'une partie d'extrémité de la partie allongée (332) de la structure, de préférence l'au moins un des premier ou second éléments de mâchoire (108, 110) ayant une extrémité effilée, et le support (182, 192) de l'ensemble de retenue (180, 190) étant disposé sur l'extrémité effilée, de préférence encore, le support (182, 192) de l'ensemble de retenue (180, 190) ayant une forme triangulaire pour réduire la flexion du matériau de renfort (310) et de la structure (330).

5. Dispositif d'agrafage chirurgical selon l'une quelconque des revendications précédentes, la partie allongée de la structure (332) s'étendant le long d'une partie périphérique du matériau de renfort (310).

6. Dispositif d'agrafage chirurgical selon l'une quelconque des revendications précédentes, la partie de mise en prise (336) de l'ensemble de renfort (300) étant fixée de manière amovible au support (182, 192) de l'ensemble de retenue (180, 190) par encliquetage ou par frottement.

7. Dispositif d'agrafage chirurgical selon l'une quelconque des revendications précédentes, la structure (330) de l'ensemble de renfort (300) étant bioabsorbable.

8. Dispositif d'agrafage chirurgical selon l'une quelconque des revendications précédentes, au moins une partie de la partie allongée (332) de la structure (330) étant interposée entre le matériau de renfort (310) et les premier ou second éléments de mâchoire (108, 110).
